**Europäisches Patentamt**

(19) **European Patent Office**

**Office européen des brevets**

(11) Publication number : **0 503 916 A1**

# (12) EUROPEAN PATENT APPLICATION

(21) Application number : **92302064.8**

(22) Date of filing : **11.03.92**

(51) Int. Cl.$^5$ : **G01N 33/569**, G01N 33/68, A61K 39/21, C12P 21/08

The applicant has subsequently filed a sequence listing and declared, that it includes no new matter.

(30) Priority : **11.03.91 US 668266**
**06.03.92**

(43) Date of publication of application :
**16.09.92 Bulletin 92/38**

(84) Designated Contracting States :
**PT**

(71) Applicant : **IDEC PHARMACEUTICALS CORPORATION**
**11099 North Torrey Pines Road**
**La Jolla California 92037 (US)**

(72) Inventor : **Chang-Yuil Kang**
**1736 Buttercup Road**
**Encinitas,CA 92024, Country of San Diego (US)**

(74) Representative : **Sexton, Jane Helen et al**
**J.A. Kemp & Co. 14 South Square Gray's Inn**
**London WC1R 5LX (GB)**

(54) **Methods for selecting antibody reagents; anti-idiotype antibodies; and aids vaccine formulations.**

(57) A novel method is disclosed for selecting antibody reagents for vaccine formulations that are capable of inducing group-neutralizing anti-HIV antibody responses in humans. Also disclosed are anti-idiotype antibodies and hybridoma cell lines capable of producing such antibodies. Further, anti-idiotype antibody-based vaccines and formulations thereof are disclosed. The methods, antibodies, hybridomas and vaccine formulations of the present invention may be useful in the treatment and prevention of HIV infection.

EP 0 503 916 A1

**FIELD OF THE INVENTION**

The present invention relates generally to the field of immunology and, more particularly, to novel methods of selecting for therapeutically useful antibodies. Further, the present invention relates to novel anti-idiotype antibodies and vaccine formulations thereof that are useful in the immunotherapy and prevention of HIV infection.

**BACKGROUND OF THE INVENTION**

It is widely accepted that the Human Immunodeficiency Virus (HIV) is the principal etiological agent of the acquired immunodeficiency syndrome (AIDS). From a clinical standpoint, depletion of $CD4^+$ lymphocytes can account for most of the severe immunologic abnormalities in AIDS patients (Fauci, et al. (1984) *Am. Int. Med.* 100:92). The tropism of HIV is dependent primarily upon a specific interaction between the gp120 envelope glycoprotein of HIV and tire CD4 receptor molecule on target cells, e.g., T lymplrocytes and macrophages, although it has been suggested that other molecules on some cells are involved in similar interactions with HIV. Beyond the primary interaction between CD4 and gp120, the following steps are postulated to be essential for HIV entry into target cells anchorage of the virus to the cell membrane; fusion with the cell membrane; and penetration of the viral elements into the cell. Any reagent that interferes with these steps is considered to block tire virus infection and is of potential therapeutic value. Such reagents under current investigation include antibodies, which are generally functionally described as "neutralizing antibodies."

Amino acid sequence analysis of various HIV isolates from a single patient as well as different patients indicates that gp120 contains hypervariable regions interspersed among highly conserved regions. This finding indicates that HIV variation in vivo can occur rapidly during the chronic infection. The degree of diversity among different strains of HIV within the gp120 molecule may be due in part to natural selection during interactions with the host immune system. Antibodies that bind to hypervariable regions of the HIV envelope generally only react with a single isolate or strain of the virus: such antibodies are termed "type-specific." At present, about 20 different strains of HIV have been identified. For example, antibodies directed against one of these hypervariable regions of gp120 known as the "V3 loop" will neutralize HIV. However, this effect has generally been type-specific; i.e., it is well documented that among anti-gp120 antibodies, those specific to the V3 loop may exert a neutralizing effect on only a single isolate or strain of HIV. Moreover, a single amino acid mutation in the V3 region allows the virus to escape neutralization by such antibodies. Although the precise role of the V3 domain in virus infection is still in question, several reports have demonstrated that antibodies to this region neutralize virus infectivity by interfering with a post-binding event (Skinner, et al. (1988) *J. Virol.* 62:4194). Whatever the V3 region's role may be in virus infection, its variability from strain to strain and high mutation frequency make it an uncertain target for immunotherapy.

Despite the considerable sequence variation and hypervariability within gp120, all known HIV isolates share a common functional characteristic-- the ability to bind to and infect $CD4^+$ cells. This observation leads to a basic premise that the CD4 attachment site on gp120 includes one or more conserved domains. Accordingly, antibodies binding to this CD4 attachment site could be expected to prevent viral infections and to interact fully with various HIV isolates via their ability to bind to conserved regions of gp120. Antibodies that bind to conserved regions of HIV and recognize multiple strains of the virus are termed "group-specific." Thus, antibodies that will neutralize multiple strains of HIV are said to be capable of group-specific neutralization. These neutralizing antibodies would play an important role in preventing viral infection (Weiss, et al. (1985) *Nature* 316:69) as well as killing HIV-infected cells via antibody-dependent cell-mediated cytotoxicity (Rook, et al. (1987) *J. Immunol.* 138:1064) and complement-mediated cytolysis. It has been well documented that antibodies specific to the conserved region exist in the sera of HIV-infected humans (Schmittman, et al. (1988) *J. Immunol.* 141:4181; Moore, J.P. (1990) *AIDS* 4:297). Therefore, an HIV vaccine which induces antibodies capable of group-specific neutralization may be effective in producing a clinical improvement in AIDS patients and preventing infection in healthy people.

Although the clinical correlation between neutralizing antibody titer and disease progression has not been well defined, recent reports suggest that the presence of maternal antibodies to gp120 correlates positively with the uninfected status of children born to seropositive mothers (Rossi, et al. (1989) *Proc. Natl. Acad. Sci.* 86:8055; Devash, et al. (1990) *Proc. Natl. Acad. Sci.* 87:3445). However, as most HIV-seropositive individuals have a high titer of anti-HIV antibodies as well as demonstrable levels of neutralizing antibodies, it remains unresolved as to why so many of these people have a rapidly progressive infection. In attempting to answer this question, Applicant has noted in the literature that high titers of type-specific neutralizing antibody in HIV-infected individuals are not sufficient to neutralize continuously emerging new isolates brought about by mutations of gp120 (Nara, et al. (1990) *In Vaccines* 90:137, Cold Spring Harbor Laboratory, Cold Spring Harbor, NY). On the other hand, titers of group-specific neutralizing antibodies in HIV-infected individuals, which are specific against conserved regions, are low. It is, therefore, Applicant's premise that HIV vaccines must be evaluated with respect to their ability to induce high titers of group-specific neutralizing antibodies.

Although the need for a HIV vaccine is clear and the objective rationally conceived, the vaccine strategies adopted have been diverse and, to date, unsuccessful. Most HIV vaccine development strategies have been based on preparations including whole or portions of the major envelope protein. Native, recombinant, synthetic, vaccinia-encoded envelope proteins and whole inactivated virus have been prepared and tested.

For example, chimpanzees were immunized with the HIV envelope protein encoded in a vaccinia virus. The animals produced both humoral (antibody) and cellular (T-cell) immune responses specific for the envelope protein; however, these primates were not protected from infectious challenge with HIV (Hu, et al. (1989) Nature 328:721).

Kennedy and colleagues, using synthetic peptides which represent conserved immunodominant epitopes of gp41, immunized chimpanzees with a peptide carrier conjugate made from the synthetic peptides (Kennedy, et al. (1989) In Vaccines 250, Cold Spring Harbor, NY). Subsequent challenge with HIV, however, showed that the animals were not protected against infection. The use of killed whole virus vaccine (Salk, J. (1987) Nature 327:473) may be able to induce protection, but to the extent these vaccines contain envelope, this protection is expected to be only type-specific and does not circumvent the problem with the envelope polymorphism (Hahn, et al. (1986) Science 23:1548).

According to a recent report (Berman, et al. (1990) Nature 345:622), immunization of primates with recombinant gp 120, induced neutralizing antibodies and elicited protective immunity against homologous strains of HIV. Still, there was no evidence that such immunization could provide protective immunity against different HIV strains or even mutated homologous isolates.

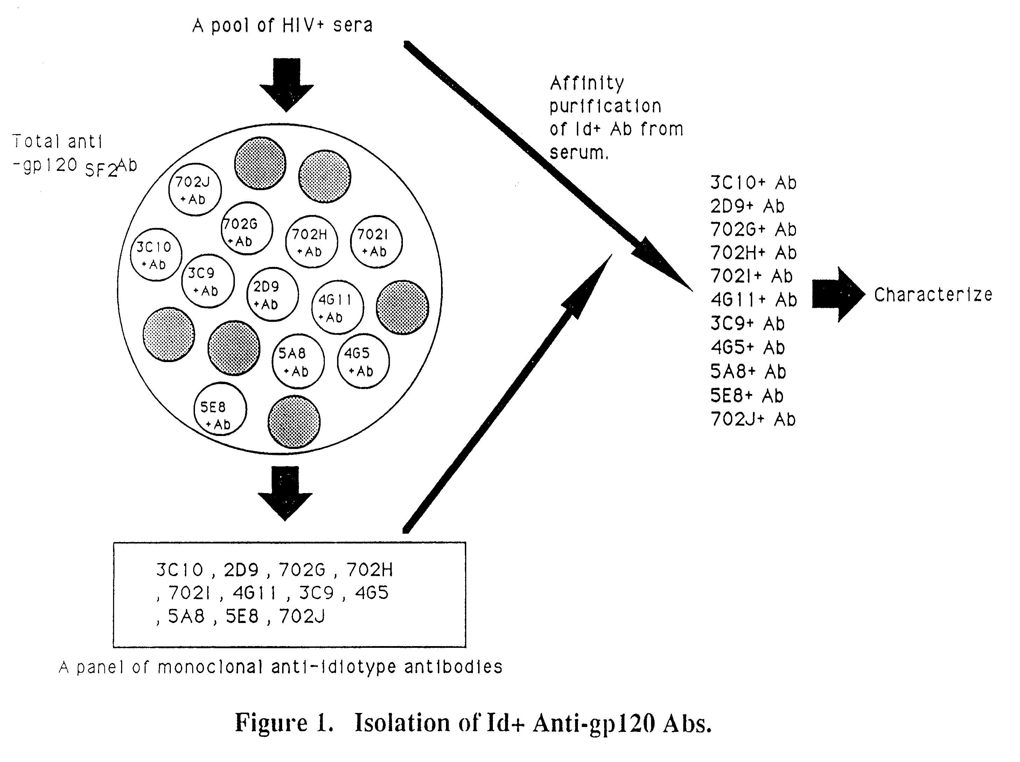

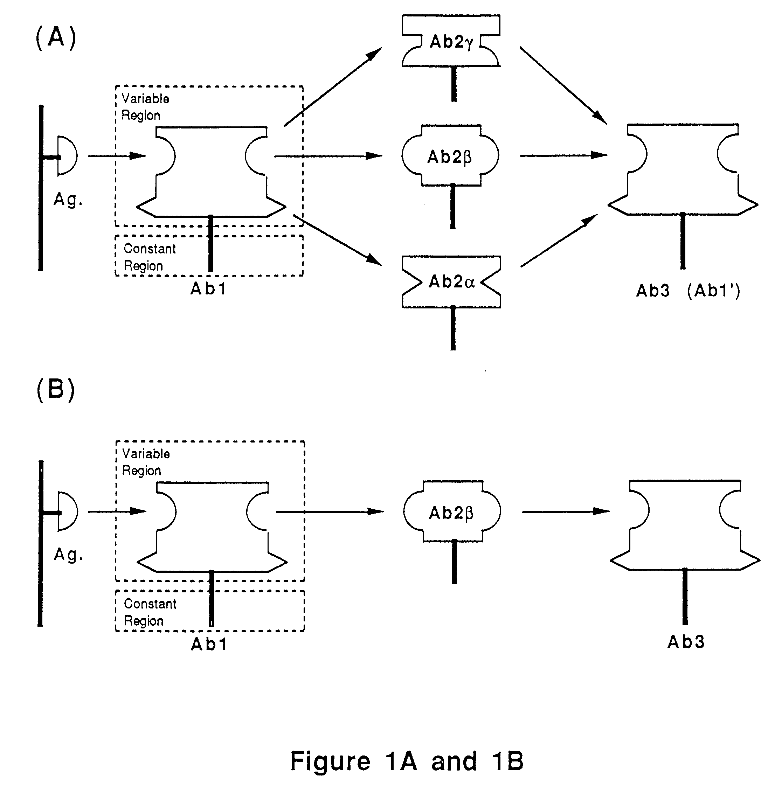

Antibody based vaccine approaches have also been investigated. For example, the use of anti-idiotype antibodies ("anti-ids") as vaccines against various infectious agents ("antigens") (so called idiotype vaccines) is well documented. To date, most idiotype vaccine development strategies have been based on the following preparations and principles. First, to generate monoclonal anti-ids (Ab2s), animal Ab1s (in most cases antibodies from non-human sources have been used) have been utilized as immunogens or templates. Second, monoclonal Ab1s have been used as templates in the majority of cases. Third, the in vitro selection of Ab2s as vaccine candidates was driven by the concept of antigen mimicry by anti-ids (Ab2s); i.e., the principle of blocking the interaction of Ab1 with Ab2 by antigen has been used as the controlling criteria for selecting Ab2 vaccine candidates. Such anti-idiotype antibodies have become know in the art as "Ab2 β " or "internal image" Ab2s. Two other types of Ab2s generated against Ab1s have been designated: Ab2 γ, which block antigen binding to Ab1, but are not internal images of the antigen; and Ab2 α, which bind to the Ab1, but do not block binding of the antigen to Ab1. See FIGURE 1. Fourth, to further define Ab2s as vaccine candidates in vivo, non-primate animals have generally been used as animal models to evaluate the Ab3 response.

Despite the tremendous financial and human resources that have been dedicated to the discovery of an effective AIDS vaccine, the results have been inconclusive at best. The reasons for these apparent failures are unclear, but some of these studies indicate that broadly reactive anti-HIV neutralizing antibodies were not generated in sufficient titer in the experimental animals, allowing HIV to "escape" the immune response.

## SUMMARY OF THE INVENTION

The present invention is based on Applicant's discovery of a novel method for selecting antibody reagents for vaccine formulations that are capable of inducing group-neutralizing anti-HIV antibody responses in humans.

In its broadest embodiment, the present invention is directed to a method of selecting monoclonal anti-idiotype antibody reagents useful in vaccine formulations for the treatment or prevention of HIV infection. The first step in this method requires preparing human polyclonal anti-gp120 antibodies (Ab1s) and subsequently generating a first group of monoclonal anti-idiotype antibodies (G1-Ab2s) capable of reacting with or immunologically binding with the human polyclonal anti-gp 120 antibodies. Then, from this first group of monoclonal anti-idiotype antibodies (G1-Ab2s), a second group or subset of monoclonal anti-idiotype antibodies (G2-Ab2s) are selected that are characterized by their ability to immunologically bind gp120 antibodies that neutralize in vitro multiple strains of HIV. Finally, the second group of monoclonal anti-idiotype antibodies (G2-Ab2s) is screened for a subset or a third group of monoclonal anti-idiotype antibodies (G3 -Ab2s) which are characterized by their ability to generate an anti-anti-idiotype antibody (Ab3) response in a primate host, wherein the anti-anti-idiotype antibody (Ab3) immunologically binds with gp 120 and neutralizes in vitro multiple strains of HIV.

In a preferred embodiment of the invention, the Ab1s are prepared from pooled human sera from HIV-positive asymptomatic individuals and purified by passing over a gp120 column to increase the concentration of anti-gp 120 antibodies. Also preferred is the generation of murine monoclonal Ab2s.

The present invention is further directed broadly to monoclonal anti-idiotype antibodies that are capable of eliciting a group neutralizing anti-HIV antibody response in humans. The present invention is also directed to monoclonal anti-idiotype antibodies (G3-Ab2s) selected according to the methods described herein.

The present invention is further directed to vaccine formulations including the monoclonal anti-idiotype antibodies (G3-Ab2s) disclosed herein.

Thus, it is an object of the present invention to develop a method for selecting antibody reagents useful in vaccine formulations which are capable of eliciting in vivo high titers of group-specific neutralizing antibodies against HIV in humans.

It is a further object of the present invention to develop antibody reagents per se that are useful in vaccine formulations and the cell lines capable of producing such antibodies.

## BRIEF DESCRIPTION OF THE DRAWINGS

FIGURE 1 graphically illustrates the concepts of "idiotype matching" (1A) and "idiotype mimicry" (1B).

FIGURE 2 shows that the flowthrough fraction of Total Anti-gp120 Abs bound to $gp120_{IIIB}$ and not to $gp120_{SF2}$.

FIGURE 3 shows that the Total Anti-gp120 Abs bound to $gp120_{IIIB}$ and not to $gp120_{SF2}$.

FIGURE 4 graphically depicts the purification of Total Anti-gp120 Abs.

FIGURE 5 illustrates the effective inhibition of $^{125}$I-labeled $gp120_{SF2}$/ CD4 by CD4-site antibodies.

FIGURE 6 depicts HIV virus neutralizing activity of CD4-site antibodies.

FIGURE 7 depicts HIV virus neutralizing activity of non-V3 antibodies.

FIGURE 8 lists the first group of anti-idiotype antibodies and the characteristics of corresponding Ab1s.

FIGURE 9 summarizes the characterization of corresponding Ab3s against candidate Ab2 vaccines in monkeys.

FIGURE 10 summarizes the neutralizing activity of Ab3 isolated from 3C9 immunized monkeys.

FIGURE 11 depicts neutralizing activity of human 3C9[+]Ab against HIV laboratory strains (*Left*) and patient primary HIV isolates (*Right*).

FIGURE 12 depicts the binding of $gp120_{SF2}$ (*Left*) and $gp120_{IIIB}$(*Right*) to purified Ab3 from monkeys PRO703 and PRO783.

## DETAILED DESCRIPTION OF THE PREFERRED EMBODIMENTS

The present invention is based on Applicant's novel anti-idiotype (anti-id or Ab2) approach to developing an HIV vaccine that possesses the critical biological property of eliciting group-specific neutralizing antibodies. Although the Applicant does not wish to be bound by any particular theory, the present invention is driven by the concept of "idiotype matching" rather than the prior art concept of "antigen mimicry" by anti-ids.

"Idiotype matching" utilizes anti-ids as surrogates for antigen, *e.g.*, in an attempt to stimulate specific elements of the B cell repertoire in humans. In contrast to the prior art, which focus exclusively on idiotype mimicry and $Ab2\beta$, the anti-id vaccine candidates of the present invention may be Ab2 $\alpha$, $\beta$, or $\gamma$ as long as they elicit group neutralizing Ab3 responses. Compare, FIGURE 1A and FIGURE 1B.

In short, most prior art anti-idiotype vaccine development strategies have been based upon antigen mimicry and the resulting principles:

(1) To generate monoclonal anti-ids, non-human Ab1s have been utilized as templates.

(2) Monoclonal Ab1s have been used as templates in the majority of cases.

(3) The *in vitro* assays used to select Ab2s as vaccine candidates have generally been based on the concept of antigen mimicry by anti-ids ($Ab2\beta$).

(4) To further define Ab2s as vaccine candidates *in vivo*, non-primates have been the animal model of choice.

Notwithstanding the scientific momentum created by these teachings in the art. Applicant developed an entirely new approach for the selection of anti-id vaccines. Some of the preferred features and advantages of Appticant's invention include the following:

(1) Human Ab1s, preferably purified from seropositive healthy individuals, are used as templates to generate Ab2s. This choice of starting material increases the likelihood that the Ab2-based vaccine will generate Ab3s in humans that are immunologically equivalent to the human Ab1s with respect to antigen binding potential, *i.e.*, anti-HIV binding. This feature of the invention takes advantage of the fact that Applicant knows that B cell repertoires pre-exists in humans that are capable of producing Ab1-like Ab3s (so called Ab1') In contrast, using a murine Ab1 assumes that the B cell repertoire of the mouse is genetically and immunologically equivalent to the human B cell repertoire-- possibly a fatal flaw for vaccine development.

(2) Polyclonal Ab1s rather than monoclonal Ab1s are used as templates to generate Ab2s. This feature of the invention eliminates the possibility that an individual monoclonal Ab1 (which represents only one epitope) selected for use as a templates does not represent a highly conserved HIV epitope.

(3) Applicant has discovered that the definition of Ab2 β and the evaluation of Ab3 responses in non-primate animals are not the critical factors in selection of an Ab2 as a vaccine candidate. In contrast, Applicant has discerned the following criteria for the selection of anti-idiotype vaccines: (a) Ab1s (which react with the Ab2 vaccine candidate) from the human sera exhibit desirable biological effects *in vitro* (*e.g.*, virus neutralization). *Ergo*, Ab3s generated *in vivo* in primates, including humans, in response to an Ab2 vaccine are expected to have Ab1-like biological effects. (b) Ab2s induce group-specific neutralizing Ab3s in primates.

The principal advantages in using Applicant's anti-id approach to select an HIV vaccine are threefold. First, selected anti-ids elicit only beneficial anti-gp120 antibodies (e.g., group-specific neutralizing antibodies). Second, anti-ids elicit high titers of certain anti-gp120 antibodies. Third, the biological properties of anti-gp120 antibodies elicited by these Ab2 are predictable.

Thus, in its broadest embodiment, the present invention is directed to a method of selecting monoclonal anti-idiotype antibody reagents that are useful in vaccine formulations for the treatment.and prevention of HIV infection. The first step in this method involves the preparation of human polyclonal anti-gp 120 antibodies (Ab1s) that are to be used as templates to generate Ab2s. It is preferred that the human polyclonal anti-gp120 antibodies are obtained from human sera of HIV-positive, asymptomatic individuals. It is further preferred that such sera is purified to increase the representation of anti-gp120 antibodies by passing the pooled human sera over a gp120 column. It is also contemplated by the present invention that the total anti-gp120 antibodies will be passed over not one but two gp120 columns, wherein each gp120 column represents a different strain of gp120. It is most preferred that the human polyclonal anti-gp 120 antibodies represent the total anti-gp120 antibodies. Alternatively, the total anti-gp 120 antibody population can be dissected into fractions for use in the present invention: CD4 attachment site-specific anti-gp 120 antibodies, V3 region-specific anti-gp 120 antibodies or non-V3 region-specific anti-gp120 antibodies.

The second step of the method of the present invention involves the generation of a first group of monoclonal anti-idiotype antibodies which are capable of reacting with the human polyclonal anti-gp120 antibodies prepared in the first step of tire method. Preferably, these anti-idiotype anti bodies are murine monoclonal antibodies generated by conventional hybridoma technology. As used herein, the term "monoclonal" is intended to encompass antibodies derived from a single clone as well as mono-specific anti bodies derived by molecular biology techniques. Alternative sources of anti-idiotype antibodies contemplated by the present invention include human, primate, chimeric, and humanized antibodies, as well as antibodies synthesized by molecular biology techniques. As used herein, the term "antibody" is intended to include whole antibodies, antibody fragments, Fab fragments, single chain antibodies, and immunologically functional anti body equivalents.

Once the first group of monoclonal anti-idiotype antibodies have been generated, the third step in the method of the present invention involves screening this first group of antibodies for a second group (subset of first group) of monoclonal anti-idiotype antibodies that are characterized by their ability to react with or immunologically bind anti-gp120 antibodies that neutralize *in vitro* multiple strains of HIV. In a preferred embodiment, the anti-gp120 HIV neutralizing antibodies are human. In a most preferred embodiment, these human anti-gp 120 HIV neutralizing antibodies are human antibodies selected from the pool of human polyclonal antibodies prepared according to the first step of the method. As used herein, neutralization of more than two strains of HIV is considered to be "multiple" strain neutralizing. These anti-gp 120 antibodies may be selected by passing the human polyclonal antibody preparation made in the first step of the method over an Ab2 (group 1) column, and selecting those anti-gp120 antibodies that are capable of *in vitro* neutralization of multiple strains of HIV.

The present invention is also directed to antibodies, in particular, to monoclonal anti-idiotype antibodies that are capable of eliciting a group- neutralizing anti-HIV antibody response in humans. Preferred are monoclonal anti-idiotype antibodies selected according to the selection method described above.

Hybridoma cell lines are also an aspect of tire present invention. In particular, hybridoma cell lines capable of producing a monoclonal anti-idiotype antibody that is capable of eliciting a group neutralizing anti-HIV antibody response in humans. The most preferred is the hybridoma cell line deposited with the ATCC, Rockville, MD on March 8, 1991, designated accession no. HB10701. In addition to the antibodies produced by this hybridoma, the present invention also includes monoclonal antibodies capable of eliciting a substantially equivalent immune response in humans as a monoclonal antibody produced by the hybridoma cell lines described above. Preferably, such a monoclonal antibody would react with and compete for binding to the same anti-gp120 antibody as a monoclonal antibody produced by the hybridoma cell lines described above.

An additional embodiment of !he invention is the development of vaccine formulations including, in mixture with a pharmaceutically acceptable carrier, a monoclonal anti-idiotype antibody that is capable of eliciting a group neutralizing anti-HIV antibody response in humans. Vaccine formulation, including selection of appropriate adjuvants and carriers, is well known to those of skill in the art. Once formulated, a vaccine's dosimetry would be based upon patient and clinical factors that are well appreciated by those of skill in tire art of vaccine delivery. The vaccines of the present invention may be either prophylactic or therapeutic in nature. Although a single anti-idiotype antibody reagent vaccine is desirable, it is contemplated by the present invention that multiple anti-idiotype antibodies may be formulated in a "cocktail" vaccine approach. Again, clinical factors will dictate the vaccine formulation and dosage. However, a preferred vaccine formulation would include about 2.5 mg of an antibody of the present invention formulated with about 100 ug of termutide and SAF (Syntex adjuvant formulation--2.0 mg polysorbate 80, 12.5 mg poloxymer 401, and 50 mg squalane). An alternative formulation may exclude the termutide component.

## EXAMPLE 1A Preparation of Starting Materials--Human Polyclonal Total Anti-gp120 Antibodies.

Total anti-gp120 antibodies (anti-gp120$_{SF2}$) ("Total Anti-gp120 Abs") were purified from human sera by affinity chromatography using a gp120$_{SF2}$-Sepharose column. Recombinant gp120$_{SF2}$ was derived from engineered CHO cells. A pool of sera from HIV-infected asymptomatic individuals (purchased from North American Biologicals, Inc., Miami, Florida 33169) were inactivated by detergent (1% NP40) and heat (1 hr. at 56°C) treatment. The sera was dialyzed against PBS and ammonium sulfate (40% as final concentration) was added to the sera to isolate the immunoglobulin fraction. The precipitated immunoglobulin fraction was reconstituted with PBS, filtered and applied to the gp120$_{SF2}$-Sepharose column. The bound Total Anti-gp120 Abs on the column were eluted with pH 3.0 glycine buffer and dialyzed against PBS. The recovery of total anti-gp120 antibodies in sera was approximately 300 µg/ml.

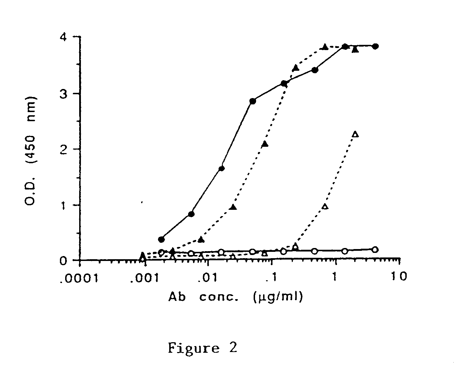

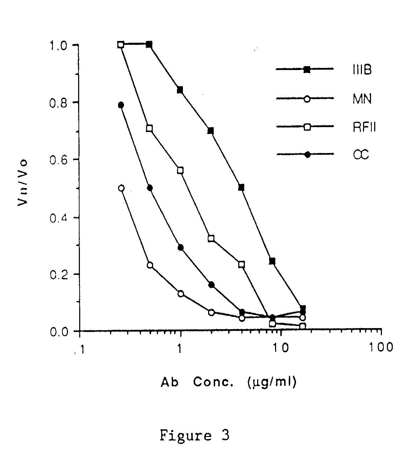

Antibodies binding to gp120$_{SF2}$ are expected to consist of two kinds: (a) $_{SF2}$ type-specific antibodies, and (b) antibodies binding to conserved regions of gp120 such as the CD4-attachment site. Since the individuals contributing to the Ig pool might have been infected with a variety of HIV strains, the flowthrough fraction may bind to gp120 derived from strains other than $_{SF2}$. Indeed as shown in FIGURE 2, Applicant found that tire flowthrough fraction bound to gp120$_{IIIB}$, not to gp120$_{SF2}$ indicating the presence of III$_B$ type-specific anti-gp120 antibodies in the flowthrough fraction. In addition, Total Anti-gp120 Abs bound to both gp120$_{SF2}$ and gp120$_{IIIB}$ (FIGURE 3) indicating the presence of a mixture of type-specific and cross-reactive anti-gp120 antibodies.

Applicant then investigated the neutralizing activities of Total Anti-gp120 Abs against various HIV isolates. FIGURE 2 shows that Total Anti-gp120 Abs exhibited neutralizing activities against four different HIV isolates, although their neutralizing activities against the different HIV isolates varied as much as 10-50 fold, indicating that Total Anti-gp120 Abs contains significantly different populations and amounts of type- and group-specific neutralizing antibodies. From these results, Applicant reasoned that the difference in the neutralizing activities of Total Anti-gp120 Abs against the various viral isolates could be explained by (a) strain-to-strain differences in the amount of anti-gp120 antibodies required for neutralization of each virion, and (b) the presence of type- or group-specific neutralizing antibodies.

To demonstrate the presence of type-specific neutralizing antibodies in Total Anti-gp120 Abs, Applicant tested the binding of Total Anti-gp120 Abs to type-specific neutralizing epitopes derived from the V3 region of different gp120s.

## Table 1

### Binding Activities of Different Anti-gp120 Antibody Preparations To Peptides Derived From the V3 Region of Various gp120s

| Anti-gp120 Antibodies | Binding Activities* | | | | |
|---|---|---|---|---|---|
| | Origin of Peptides | | | | |
| | MN | IIIB | RF | SC | WMJ-2 |
| Total Anti-gp120 Abs | >3 | 1.05 | 0.06 | 0.8 | 1.48 |
| CD4-site Abs | 0.15˙ | 0.01 | 0.01 | 0.03 | 0.01 |

*Binding activities are expressed by O.D. obtained at 5 ug/ml of each preparation.

Table 1 shows that Total Anti-gp120 Abs bind extensively to tire MN isolate derived epitope, less extensively to tire IIIB, SC and WMJ-2 derived epitopes and weakly to the rF-derived epitope. Thus, the highest binding activities of Total Anti-gp120 Abs against the MN-derived epitope is consistent with the fact that Total Anti-gp120 Abs exhibited the highest neutralizing activities against the MN isolate. However, it is interesting to note that the binding activity of Total Anti-gp120 Abs to the RF derived epitope was only slightly above background, despite the fact that Total Anti-gp120 Abs effectively neutralized the RF isolate. This could be explained by the presence of group-specific neutralizing antibodies specific against non-V3 epitopes.

**EXAMPLE 1B Preparation of CD4 Site Attachment Site-Specific Polyclonal Anti-gp120 Antibodies from Human Sera**

Isolation and Characterization of CD4 Attachment Site-Specific Anti-gp120 Antibodies

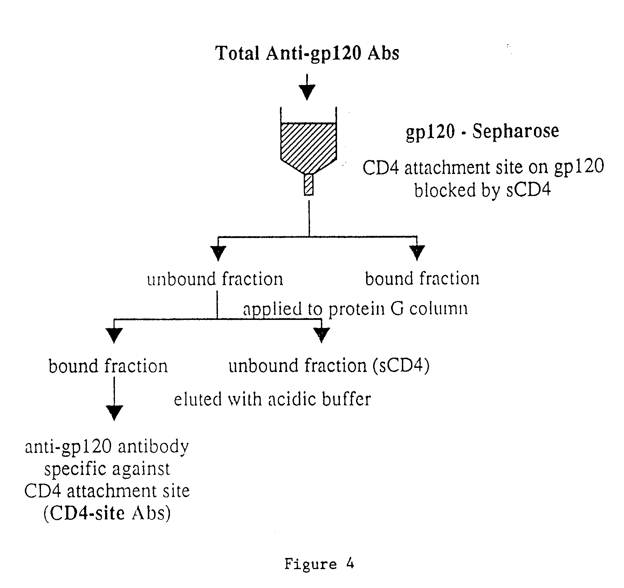

Since Applicant observed group-specific neutralizing activities with Total Anti-gp120 Abs, it was of interest to determine the epitope specificity of anti-gp120 antibodies which are responsible for these group-specific neutralizing activities. Since antibodies to the conserved CD4 attachment site of gp120 are considered to be the major group-specific neutralizing epitope, Applicant devised the following strategy. The approach to isolate CD4 attachment site-specific anti-gp 120 antibodies (CD4-site Abs) from Total Anti-gp120 Abs consisted of saturating the CD4-attachment site on gp120 immunoabsorbent with sCD4 and followed by applying Total Anti-gp120 Abs to the immunoabsorbent in the presence of excess sCD4. The flowthrough fraction of the antibodies were collected and applied to a protein G column to isolate the IgG fraction from sCD4. This is graphically depicted in FIGURE 4. To ensure complete removal of the sCD4 from the IgG fraction, Applicant analyzed this preparation by SDS gel electrophoresis and ELISA, using a monoclonal anti-CD4 antibody, and could not detect any sCD4 by either method.

To isolate the CD4 attachment site-specific anti-gp120 antibodies, 10 mg of sCD4 in PBS was repeatedly loaded on tire gp120$_{SF2}$-Sepharose column to saturate tire CD4 attachment site onto the gp120. Then, 4 mg of Total Anti-gp120 Abs with 10 mg of sCD4 in PBS was loaded onto tire column. The flowtlrrough fraction was further separated on a protein G column (Pharmacia Fine Chemicals, Piscataway, NJ) to isolate the IgG fraction. SDS gel electrophoresis and ELISA, using a monoclonal anti-CD4 antibody, were employed to verify that the isolated IgG was free of sCD4.

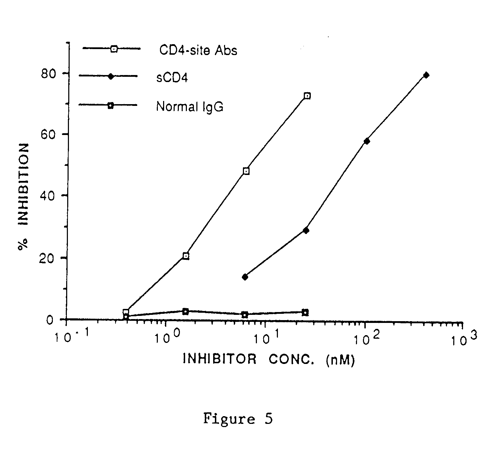

To examine whether the IgG fraction contains CD4-site Abs, Applicant performed competitive inhibition assays on CD4[+] cells using $^{125}$I-labeled gp120$_{SF2}$ and CD4-site Abs. FIGURE 5 shows that CD4-site Abs effectively inhibited binding of $^{125}$I-labeled gp120$_{SF2}$ to CD$_4$ cells. The approximate IC$_{50}$ of CD4-site Abs in this assay was ten times less than that of sCD4. Applicant observed almost the same results with $^{125}$I-labeled gp 120III$_B$.

This result indicated that this IgG fraction contained significant amounts of CD4-site Abs. To determine what proportion of this IgG fraction are CD4-site Abs, various anti-gp120 antibodies were coated on micotiter plates and incubated with $^{125}$I-labelled gp120$_{SF2}$ in the presence or absence of sCD4.

## Table 2

### Proportion of CD4 Attachment Site-Specific Antibodies in Different Anti-gp120 Antibody Preparations

| Anti-gp120 Antibodies | Proportion* as % |
|---|---|
| Total Anti-gp120 Abs | 16 |
| CD4-site Abs | 71 |
| Non-V3 Abs | 32 |
| V3-Region Abs | 0 |

*The percent inhibition by sCD4 in each anti-gp120 antibody binding to gp120 was calculated. The largest percent inhibition was considered to be the proportion of CD4 attachment site-specific anti-gp120 antibodies.

Table 2 shows that sCD4 inhibited the binding of CD4-site Abs to gp120$_{SF2}$ by 71%, whereas only 16% of Total Anti-gp120 Abs binding to gp120$_{Sf2}$ was inhibited by sCD4. This result indicated that our purification significantly enriched CD4-attachment site-specific anti-gp120 antibodies; however, these antibodies still contained substantial amounts of non-CD4 attachment site-specific anti-gp120 antibodies. In an attempt to determine the specificities of the contaminating antibodies in CD4 site Abs, Applicant tested the binding of CD4 site Abs to synthetic peptides derived from the V3 domain of different HIV isolate gp120s. Table 1 shows that CD4 site Abs bound only weakly to the MN isolate-derived epitope and not to the other isolate-derived epitopes. This finding demonstrates that the purification for CD4-site Abs removes most of the V3 region-specific anti-gp120 antibodies.

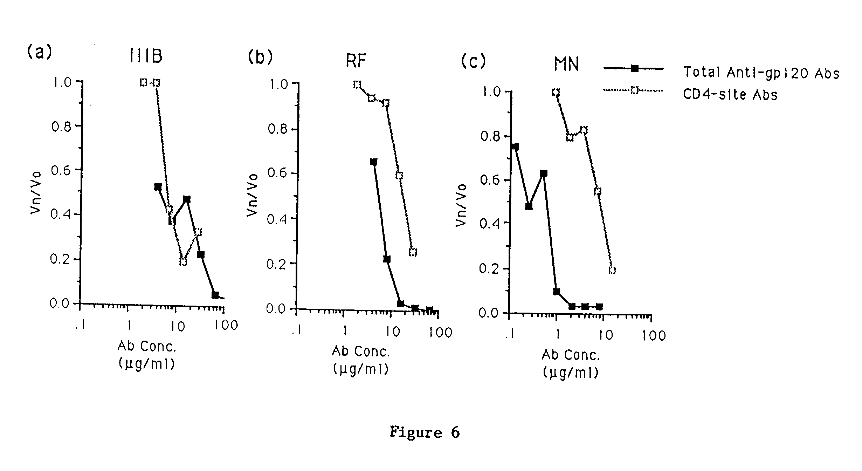

Since Applicant found that CD4-site Abs contain mainly CD4-attachment site-specific anti-gp120 antibodies with minimal amounts of MN isolate specific neutralizing antibodies, Applicant investigated whether CD4-site Abs could neutralize different HIV isolates. As shown in FIGURE 6, CD4-site Abs exhibited almost the same extent of neutralizing activities against three different HIV isolates. Compared with CD4 site Abs, Total Anti-gp120 Abs exhibit much higher neutralizing activities against the MN isolate, a little higher against the RF isolate and almost the same against the III$_B$ isolate. From the fact that CD4 site Abs effectively neutralized three different HIV isolates at or near equivalent antibody concentrations despite low or no binding activities against identical V3 specific peptides, Applicant concluded that CD4-site Abs exhibit mainly non-V3 group-specific neutralizing activities, whereas, Total Anti-gp120 Abs exhibit type- and group-specific neutralizing activities. Applicant utilized these CD4-site Abs to generate murine monoclonal anti-ids.

### EXAMPLE 1C. Preparation of Anti-gp120 Antibodies Depleted of Type-Specific Neutralizing Antibodies

Another approach for identifying group-specific neutralizing antibodies was to deplete type-specific neutralizing antibodies from TAGA, using a column of V3 peptide Affi-Gel 401 and test this preparation for group-specific neutralizing activities.

To separate the anti-gp120 antibodies depleted of V3 region-specific antibodies (non-V3 Abs) from Total Anti-gp120 Abs, a mixture of two peptides (8 mg of each) derived from the V3 region of gp120$_{SF2}$ (see peptides for sequence) were coupled to 10 ml of Affi-Gel 401 (Bio-Rad, Richmond, CA). See method below. The peptides were synthesized on an automated peptide synthesizer (model 430A, Applied Biosystems, Foster City, CA) by solid phase method. The peptides were deprotected and cleaved from the support with HF/anisole or the "low-high" HF procedure. The sequences of two SF2-derived peptides for an affinity column were as follows; V3SF2: TRPNNNTRKSIYIGPGRAFHTTGRIIGDIRKAHC and CTRPNNNTRKSIYIGPGRAFHTTGRIIGDIRKAHC. The sequences of various V3 region peptides for binding assays were as follows;

MN: YNKRKRIHIGPGRAFYTTKNIIG, IIIB: NNTRKSIRIQRGPGRAFVTIGKIG, RF: NNTRKSITKGPGRVIYATGQIIG, SC: NNTTRSIHIGPGRAFYATGDIIG, WMJ-2: NNVRRSLSIGPGRAFRTREGN. Ten mg of Total Anti-gp120 Abs in PBS was repeatedly loaded on the peptide Affi-Gel column. The flowthrough, consisting of anti-gp120 antibodies depleted of V3 region-specific antibodies (non-V3 Abs), was collected. SF2 V3 region-specific anti-gp 120 anti bodies (V3-region Abs) were eluted with pH3 glycine buffer.

Peptide Coupling

1. Derivatization of tire Affinity Support

An aliquot of Affi-Gel 401 affinity support was reduced with 100 mM dithiothreitol for 1 hour at room temperature. The resin was then transferred to a sintered glass funnel and washed extensively with argon-saturated phosphate buffered saline.

The reduced resin was then transferred to a 15 ml conical tube and diluted with 10 ml of argon-saturated phosphate buffered saline. The bis-maleimidomethyl ether in ethanol was added to the resin in a 100 fold molar excess of maleimide over sulfhydryls. The reaction was allowed to proceed overnight at 1-8°C with rotation in a sealed tube. The resin was subsequently transferred to a sintered glass funnel and washed extensively with argon-saturated PBS in order to remove excess reagent. Umeacted sulfhydryls were blocked by the addition of n-ethylmaleimide in ethanol in a sealed tube with rotation at 2-8°C overnight.

2. Reduction of peptide and Coupling to the Derivatized Support

Reduction of the C terminal cysteine was effected by the addition of 100 mM DDT with incubation for 2 hours at room temperature. The reduced peptide was separated from reductant by adsorption to a Sep-Pak C-18 apparatus, washed with argon-saturated PBS, and eluted directly into tire support in solution with 95% CH$_3$CN. Coupling was allowed to take place overnight at 2-8°C with rotation in a sealed tube. Following the coupling, tire resin was washed with PBS in order to remove excess peptide.

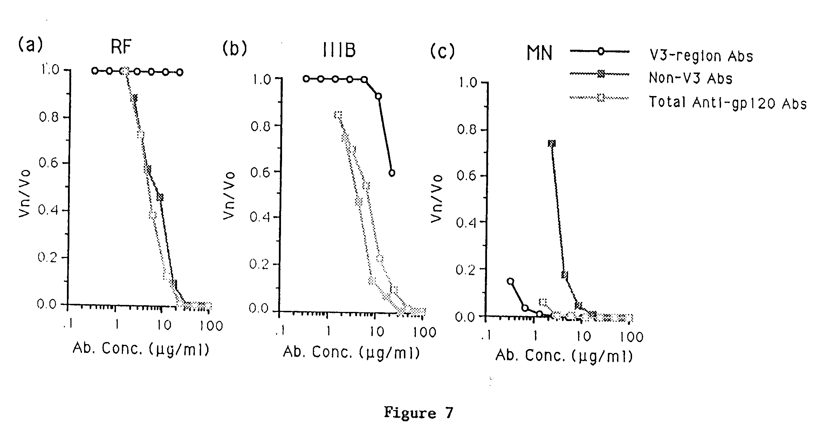

Both bound and flowthrough fractions were tested in virus neutralization assays. FIGURE 7 shows that non-V3 Abs exhibited almost the Same extent of neutralizing activities against three different HIV isolates. However, V3 region Abs exhibited strong neutralization activities against the MN isolate, minimal activities against IIIB and no activity against RF. Based on these observations, Applicant concluded that the neutralizing activities of non-V3 Abs are group-specific while the neutralizing activities of V3 region Abs are type-specific. This result indicates that the majority of neutralizing activity of Total Anti-gp120 Abs to III$_B$ and RF isolates (FIGURES 7a, 7b) are derived from group-specific neutralizing activities of the non- V3 Abs fraction, whereas, most of the neutralizing activity of Total Anti-gp120 Abs to the MN isolates (FIGURE 7c) are derived from type-specific neutralizing antibodies in the V3 region Abs fraction. Furthermore, by comparison of neutralizing activities of V3 region Abs and non-V3 Abs on MN isolate (FIGURE 7c), Applicant concluded that V3 region Abs are more effective than non-V3 Abs neutralizing antibodies in neutralizing a specific isolate.

Conclusions on Preparation of Anti-gp120 Antibodies in Examples 1A. 1B, and 1A

Applicant has dissected the neutralizing activities of various polyclonal anti-gp120 antibodies present in a pool of serum from 4 HIV-infected individuals to understand the role of these antibodies in the host defense against HIV infection. The major findings of this study are as follows: (i) Total Anti-gp120 Abs exert type- and group-specific neutralizing activities; (ii) the group-specific neutralizing activity is found primarily in CD4-site Abs; (iii) if other group-specific neutralizing antibodies rather than CD4-site Abs exist, they are not specific against a simple linear epitope of the V3 domain; (iv) V3 region Abs are more effective than non- V3 Abs in neutralizing a specific HIV isolate.

Although neutralizing efficacy of CD4-site Abs is relatively low, it is reasonable to assume that an HIV vaccine which induces high titers of CD4 site Abs should be effective in producing a clinical improvement in AIDS patients and preventing infection against various HIV isolates in heal thy people.

## EXAMPLE 2A Generation of First Group of Anti-id Mabs and Characterization for their Ability to React with Human Polyclonal Anti-gp120 Antibodies.

Generation of anti-id Mabs was accomplished by fusing sp2 murine myeloma cells with splenocytes from mice which were immunized 2-3 times with polyclonal anti-gp 120 antibodies. In screening assays, anti-id was selected for (i) binding to polyclonal human anti-gp120 antibodies, (ii) not binding to any specific isotype standard antibody, (iii) no binding to Ig preparation of the same pool of sera depleted of Total Anti-gp120 Abs by absorption on a $gp120_{SF2}$-Sepharose column.

### Immunizations

BALB/c mice were immunized with TAGS and the various other fractions of polyclonal anti-gp120 antibodies purified from a pool of four sera from HIV-infected individuals as described in EXAMPLES 1A-C. All immunizations were done by two S.C. injections of 50 micrograms of antibody in SAF-MDP adjuvant at two week intervals, followed by a single i.p. injection of 50 micrograms of antibody in incomplete Freunds adjuvant ("IFA") 3 days prior to fusion. Spleen cells were fused with SP2 myeloma cells and hybrids were selected in HAT medium. Culture supernatant were tested by ELISA. positive clones were subcloned twice. Anti-id Mabs were purified from the ascites on protein A-Sepharose columns.

### ELISA Assay for Hybridoma Screening

For hybridoma screening assays, microtiter wells were coated with 300 ng of tire following human Igs in PBS: Different anti-gp120 antibodies used for immunizations; Ig preparations of the same pool of sela depleted of Total Anti-gp120 Abs by absorption on a $gp120_{SF2}$-Sepharose column; and myeloma proteins representing all different isotypes of human heavy and light chains (The Binding Site, San Diego, CA). Culture supernatants were tested for their binding activities to the antibody coated plates. Alkaline phosphatase-coupled goat anti-mouse Ig and phosphatase substrate (Sigma, St. Louis, MO) were used to detect antibodies binding to coated plates.

### EXAMPLE 3A. SELECTION OF A SECOND GROUP OF ANTI-IDS FROM THE FIRST GROUP OF ANTI-IDS BASED ON *IN VITRO* CHARACTERIZATION

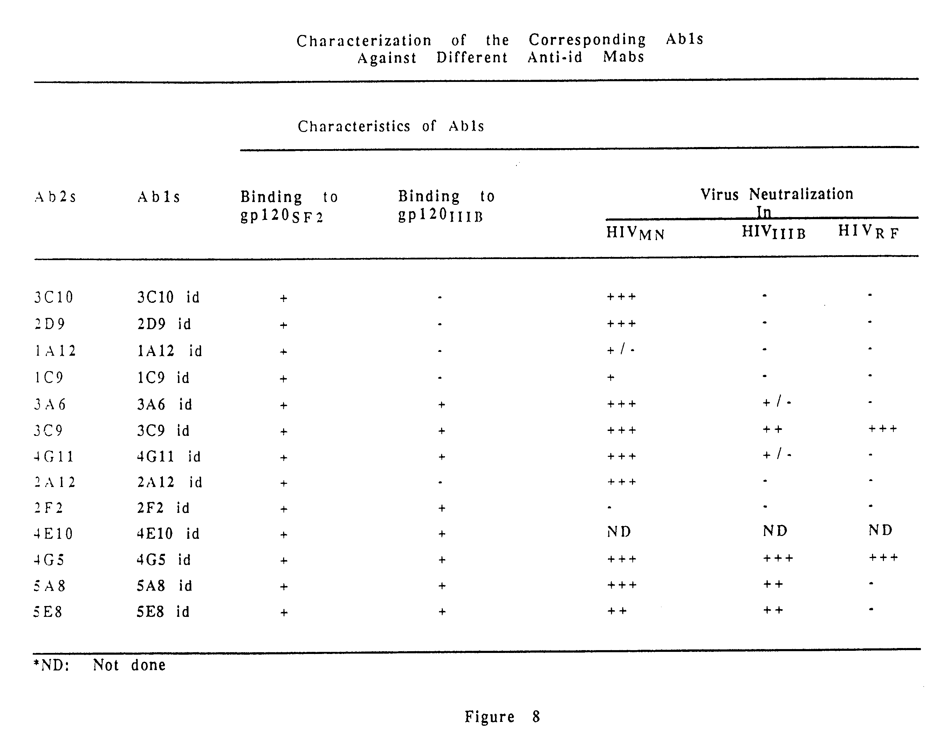

Characterization of anti-id Mabs was necessary to identify appropriate antibodies for tire next level of experiments. For this procedure, a unique approach to define Ab2s as vaccine candidates *in vitro* was employed. The underlying principals are based on "idiotype matching" rather than the antigenic mimicry of anti-id (Ab2β). Therefore, the isolation of the corresponding Ab1s to individual Ab2s from human sera and their evaluation was a crucial step. Thus, Applicant isolated the corresponding Ab1s using an Ab2-Sepharose column and tested the Ab1s for binding activity to gp120 and neutralizing activity against multiple strains of HIV. Then, Ab2s which interact with group-specific neutralizing Ab1s were selected for primate studies. See FIGURE 8.

### Preparation of Id+Abs (Ab1s)

Anti-id Mabs were conjugated to Sepharose 4B by mixing 8 mg of antibodies with 5 ml of CNBr-activated Sepharose 4B (pharmacia, piscataway, NJ). Then, 20-60 ml of the same sera pool used for various anti-gp120 antibody preparation was loaded to anti-id Mab-Sepharose 4B columns. After washing extensively, bound antibodies (Ab1 Ids) were eluted with citrate buffer ph2.8 and dialyzed against PBS.

### ELISA

An ELISA plate was coated overnight with 0.2 ml of 0.5 μg/ml of $gpl120_{SF2}$ or $gp120III_B$ (SmithKline Beecham, king of Prussia, PA) in 0.1 M bicarbonate buffer pH 9.8. The plate was washed three times with PBS and blocked for 1 hr. with 10% FCS in PBS. After washing, various concentrations of Ab1s were added. Two hours later, the plate was washed and biotin-coupled goat anti-human Ig and avidin-coupled peroxidase was used for detecting antibody binding to tire ELISA plate.

### Neutralization assay

A quantitative neutralization assay of HIV by anti-gp 120 antibody was conducted as previously described (Nara, *et al.* (1988) *Nature* 332:469). The virus stocks of HIV isolates were grown in H9 cells and harvested for optimal viral infectivity and aliquoted and frozen at -140°C in :a vapor phase cryo unit. Equivalent amounts of virus (syncytial-forming unit) were used in the assays so as to allow for comparison of the various antibody fraction tested. The assays are repeatable over a virus surviving fraction range of 1 to 0.001 within a two to four fold dilution of tire antibody ($p \leq 0.001$).

## EXAMPLE 4A. SELECTION OF THIRD GROUP OF ANTI-IDS FROM THE SECOND GROUP BASED ON AB3 RESPONSE IN PRIMATES

### Demonstrations of HIV Vaccine Activity of Anti-Id Mabs

To identify anti-id Mabs with potential utility in eliciting group-specific neutralizing anti-gp120 anti bodies, cynomolgus monkeys were used. Monkeys were immunized with anti-id Mab/Syntex Adjuvant Formulation ("SAF") (Syntex Corp., Palo Alto, CA) emulsion, each given at two week intervals. After the fourth immunization, blood samples were taken and sera were isolated and used to prepare Ab3 preparation on Ab2 affinity column. Applicant next examined whether Ab3 binds to gp120.

### Ab3 binding to gp120

To test gp120 binding activity of Ab3, $gp120_{SF2}$ and $gp120_{IIIB}$ were iodinated by Bolton-Hunter reagent (ICN Biochemical, Cleveland, OF) and lactoperoxidase (Bio-Rad, Richmond, CA) respectively. Microtiter plates were coated for 18 hours with 1 ug of Ab3 in PBS. After washing, the plates were blocked for one hour with 1% BSA in PBS, 10,000 cpm of $gp120_{SF2}$ or $gp120III_B$ were added to wells and incubated for 3 hours. After washing three times, each well was counted.

### HIV Neutralization

For neutralizing assays, Ab3 was further purified on gp120-Sepharose affinity column to enrich gp120 specific Ab3s. Applicant then tested whether gp120 specific Ab3s neutralize multiple strains of HIV. Two quantitative neutralization assays of HIV were performed, as previously described (Nara, *et al.* (1988) *Nature* 332:469). Briefly, target cells of the CEM-SS cell line were infected in monolayers by different HIV isolates grown in H9 cells, in tire presence of Ab3s. After 7-12 days in culture, syncitia forming units were counted, md supernatants were assayed in ELISA for p24 concentration.

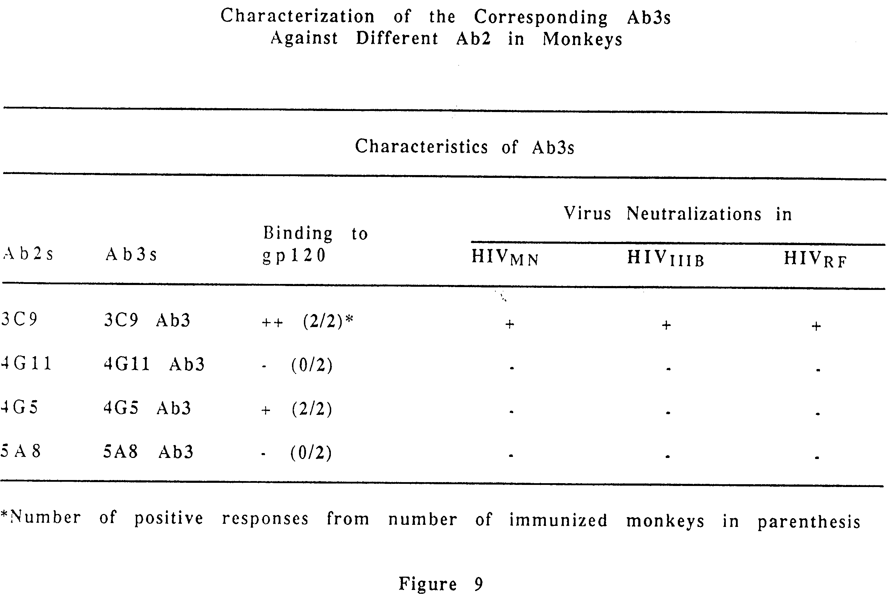

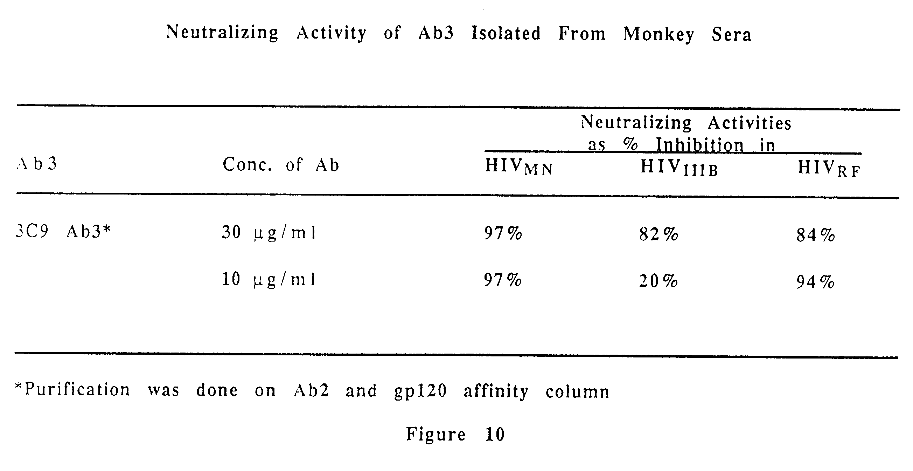

As shown in FIGURES 9 and 10, Applicant found that Ab3s isolated from the sera of 3C9 immunized monkeys bind to gp120 and the purified gp120-specific Ab3s neutralize three distinctive HIV isolates.

## EXAMPLE 4B. CHARACTERIZATION OF 3C9 AS ACTIVE IMMUNOTHERAPY REAGENT

### Immunizations

Four Cynomolgus monkeys received intramuscular injections with 2.5 mg of 3C9 in 1 ml of SAF (Syntex adjuvant formulation) containing 0.6 mg of N-acetylmuramyl-L-alanyl-D-isoglutamine (MDP). The injections were administered bi-weekly. Animals were bled before the injection series and seven days after each injection.

### Recombinant Proteins

$gp120_{IIIB}$ is a recombinant glycoprotein of an $HIV_{IIIB}$ isolate which is secreted from a *Drosophilia* cell line. $gp120_{SF2}$ is a recombinant envelope glycoprotcin of an $HIV_{SF2}$ isolate which is secreted from Chinese hamster ovary (CHO) cells. The $gp120_{IIIB}$ and $gp120_{SF2}$ are readily available to those of skill in the art. Recombinant soluble CD4 (sCD4) was prepared by transfection of a truncated CD4 gene into CIIO cells. Secreted sCD4 in culture supernatants of CHO cells (representing 95% of the amino-terminal extracellular portion of the molecule) was purified on an anti-CD4 antibody conjugated Sepharose column.

## Affinity Purification of Antibodies

All affinity columns were prepared by conjugating 8 mg of protein with 5 ml of CNBr-activated Sepharose 4B (Pharmacia). To purify the 3C9-reactive antibody (3C9$^+$Ab) from pooled sera of healthy HIV-infected individualy (Kang, *et al.* (1991) *Proc. Natl. Acad. Sci. USA* 88, 6171-6175), heat (56°C, 1 hr)- and detergent (1% Nonidet P-40)- treated sera were applied to a 3C9-conjugated Sepharose affinity column. After being washed extensively, bound antibodies (3C9$^+$ Abs) were eluted with citrate buffer (pH 2. 8) and dialyzed against phosphate-buffered saline (PBS). To purify various Ab3s from monkey sera, all 3C9 immune sera were initially diluted 1:1 with PBS. The diluted serum was then passed over an affinity column of normal mouse immunoglobulin-conjugated Sepharose. The flow-through fraction was collected and set aside. After washing, the bound antibody (anti-isotype Ab) was washed, eluted with citrate , buffer, and dialyzed against PBS. Subsequently, the Ab3 was passed over a gp120$_{SF}$-Sepharose affinity column, and the column was washed extensively. The bound antibody (gp120-specific Ab3) was eluted with citrate buffer and dialyzed against PBS.

## RIA

gp120$_{SF2}$ and gp120$_{IIIB}$ were labeled with $^{125}$I by using a Bolton-Hunter Reagent Kit (NEN). RIA plates (Dynatech) were coated for 18 hr with various amounts of antibody in PBS. The plates were washed and then blocked for one hr with PBS containing 1 % bovine serum albumin (BSA). $^{125}$I-labeled gp120$_{IIIB}$ or gp120$_{SF2}$ in PBS containing 10% (vol/vol) fetal calf serum and 0.05% Tween 20 was added in the presence or absence of different concentrations of inhibitors. After 3 hr, the plates were washed with PBS and the bound radioactivity was determined in a gamma counter.

## ELISA

To test the binding activity of 3C9$^+$Abs to a V3 region-derived peptide (amino acids 301-338 of gp120$_{SF2}$), microtiter plates were coated overnight with 0.25 ug of protein in PBS per well. After the adherent layer was blocked and washed, different concentrations of antibody in PBS were added to the plates. Alkaline phosphatase-coupled goat anti-human immunoglobulin and phosphatase substrate (Sigma) were used to detect antibodies binding to the plates.

## Neutralization Assays

Two quantitative neutralization assays with HIV-1 laboratory stains were performed, as previously described (Nara & Fischinger. (1988) Nature (London) 332. 469-470: Whalley *et al.* (1992) *Viral Immunol.*, 4, 201-213). Briefly, monolayers of CEM-SS target cells were cultured with different HIV strains, in tire absence or presence of different neutralizing antibodies. After 3-5 days in culture, syncytia-forming units were counted and in the same assay, supernatants were assayed in ELISA for p24 concentration 7-12 days later. Neutralization assays with HIV primary isolates were performed as described (Daar *et al.* (1990) *Proc. Natl. Acad. Sci.* USA 87, 6574-6578).

## Property of Anti-Idiotypic mAb 3C9 as Candidate for Clonotypic Stimulation

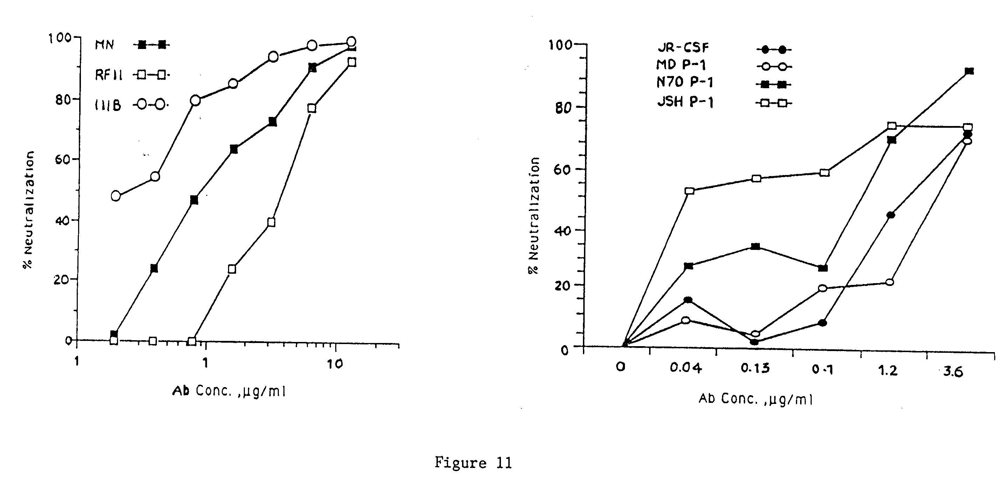

Using the methods described above, Applicant generated a series of anti-idiotypic antibodies against purified human anti-gp120 antibodies from a pool of four healthy HIV-1 infected donors. Briefly, anti-gp120 antibodies were purified on gp120$_{SF2}$-Sepharose and used to immunize mice for hybridoma fusion. Anti-idiotype mAbs were selected for binding to human anti-gp120 antibodies. One anti-idiotype mAb, 3C9, with specificity for CD4 site-directed anti-gp120 antibodies, was further characterized as a candidate for induction of anti-gp120 antibodies. Pooled human HIV-1$^+$ sera were purified on a 3C9 immunoabsorbent and eluted antibodies (3C9$^+$Ab) were tested for binding to gp120 and V3 loop peptides. The results (Table 3), show that 3C9$^+$Ab bound to two genetically distinct gp120s derived from HIV$_{SF2}$ and HIV$_{IIIB}$ and did not bind to the V3 loop peptide. Furthermore, 3C9$^+$Ab binding to gp120 was inhibited by sCD4, indicating that 3C9$^+$Ab is specific for an epitope around the CD4 attachment site of gp120. Next, the biological function of 3C9$^+$Ab was examined in virus neutralization assays. Human 3C9$^+$ Ab exhibited different spectra of broadly neutralizing activities against genetically distinct HIV laboratory strains and four primary isolates established from HIV-infected individuals. See Fig. 11. Collectively, these results indicate that anti-idiotype mAb 3C9 recognizes a clonotypic determinant shared by human polyclonal anti-gp120 antibodies that are specific for a conserved epitope around the CD4 attachment site and exhibit broadly neutralizing activities. By this definition, 3C9 defines a marker on B cells producing neutralizing

anti gp120 antibodies.

**Table 3.**

Immunological Properties of Human 3C9-Reactive Antibodies (3C9+ Ab)

| | Binding activity of 3C9± Ab in assays | | | | |
|---|---|---|---|---|---|
| | cpm in RIA with $^{125}$I-gp120† | | | | A in ELISA with V3 loop peptide†† |
| | gp120$_{SF2}$ | | gp120$_{IIIB}$ | | |
| Ab coating* | Without sCD4 | With sCD4 | Without sCD4 | With sCD4 | |
| 3C9+ Ab | 5058 | 1539 | 8394 | 1963 | 0.07 |
| Normal hIg | 145 | 151 | 282 | 278 | ---- |
| V3-specific hAb (polyclonal) | ---- | ---- | ---- | ---- | 1.77 |

†Plates were coated with 0.3 ug of antibody per well. After addition of PBS containing 10% fetal calf serum to block the adsorbed layer, $5 \times 10^4$ cpm of $^{125}$I-labeled gp120$_{SF2}$ or $^{125}$I-labeled gp120$_{IIIB}$ was added to each well in the presence or absence of sCD4 at 50 ug/ml. After 3 hr, the plates were washed and the radioactivity was measured.

*3C9+ Ab was purified from HIV+ human sera on a 3C9-conjugated Sepharose column, normal human immunoglobulin (hIg) was from The Binding Site (San Diego), and V3-specific human polyclonal antibodies (hAb) were purified from HIV+ human sera on a V3$_{SF2}$-conjugated Affi-Gel column as described (Kang *et al.* (1991) *Proc. Natl. Acad. Sci USA* 88, 6171-6175).

††Plates were coated with 0.25 mg of V3$_{SF2}$ peptide in PBS per well. After addition of PBS containing 1% BSA to block the adsorbed layer, 1 ug of antibodies per ml of PBS was added to the plates. Alkaline phosphatase-coupled goat anti-human Ig and phosphatase substrate were used to detect antibodies binding to the plates. Absorbance was measured at 405 nm.

### 3C9 mAb Induces Anti-gp120 Ab3 Antibodies

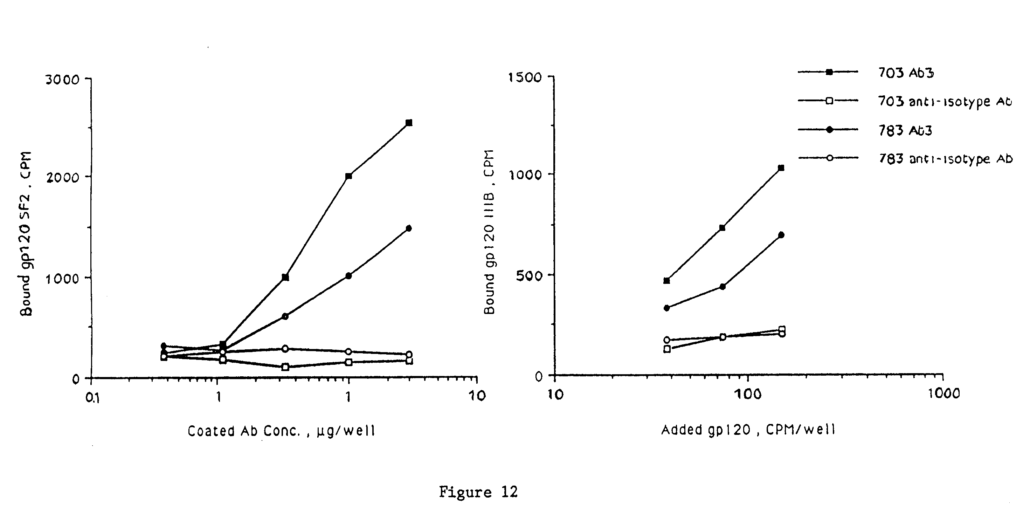

To test whether 3C9 elicit anti-gp120 antibodies, four cynomolgus monkeys were immunized with 3C9 mAb in Syntex adjuvant formulation. Since the immune serum was expected to contain 3C9-specific antibodies (Ab3) and mouse immunoglobulin-specific antibodies (anti-isotype Ab), it was fractionated on successive affinity columns and tested for binding to gp120. Ab3 from two monkeys bound to gp120$_{SF2}$ in an antibody concentration-dependent manner and to gp120$_{H4B}$ in an antigen concentration-dependent manner, whereas anti isotype Abs did not bind to either type of gp120. See Fig. 12. Similar results were obtained with Ab3s from two other monkeys (data not shown). Inhibition assays were performed to analyze the specificity of Ab3. The results in Table 4 show that gp120 binding of Ab3 from monkeys PRO703 and PRO783 were inhibited by 3C9 anti-idiotype mAb (Ab2), human 3C9+Ab (Ab1), and sCD4. Similar results were obtained with Ab3s from other monkeys (data not shown). These results confirm the anti-clonotypic nature of 3C9 as it is able to induce in primates gp120-specific Ab3 with similar specificity to the human anti-gp120 antibodies (3C9+Ab) purified on 3C9 immunoabsorbent (see Table 3).

Table 4.

Inhibition of Ab3 Binding to gp120$_{SF2}$ by Various Inhibitors

| Monkey Ab3 binding to gp120$_{SF2}$ | Inhibitor | Conc., ug/ml | %inhibition |
|---|---|---|---|
| PRO703 Ab3 | 3C9 | 5 | 94 |
| | 3C9$^+$ Ab* | 5 | 98 |
| | sCD4 | 30† | 73 |
| | BSA | 100 | 2 |
| PRO783 Ab3 | 3C9 | 5 | 95 |
| | 3C9$^+$ Ab* | 5 | 97 |
| | sCD4 | 30† | 79 |
| | BSA | 100 | 0 |

Plates were coated with 2 ug of antibody per well. After addition of PBS containing 10% fetal calf serum to block the adsorbed layer, 1.5 x 10$^5$ of $^{125}$I-labeled gp120$_{SF2}$ was added to each well in the presence or absence of inhibitors. After 3 hr, the plates were washed, the radioactivity was assayed, and the percent inhibition was calculated.

*3C9$^+$ Ab was purified from HIV$^+$ human sera on a 3C9-conjugated Sepharose column.

†At this concentration, sCD4 did not inhibit V3 region-specific antibody to gp120$_{SF2}$, as previously described.

Monkey Ab3 antibodies were further fractionated on a gp120$_{SF2}$-Sepharose affinity column. The flowthrough and bound fractions were collected as non-gp120-specific Ab3 and gp120-specific Ab3, respectively. These antibodies were then examined for gp120 binding activity. The data in Table 5 shows that all gp120-specific Ab3 from four monkeys bound to gp120, whereas non-gp120-specific Ab3 did not. This finding indicates that only a fraction of Ab3 recognized gp120 and also suggests that the gp120-specific Ab3 from all four monkeys had a range of affinities for gp120. For example, gp120-specific Ab3 of PRO703 exhibited a 3 to 4-fold higher binding to gp120 than that of gp120-specific Ab3 from the other three monkeys.

Table 5.
Binding of gp120-Specific Ab3 Antibodies to gp120

| Monkey | Ab3, ug per well | Binding of $^{125}$I-labeled gp120$_{SF2}$ to Ab3, cpm | |
| | | gp120-specific Ab3 | non-gp120 specific Ab3 |
| --- | --- | --- | --- |
| PRO703 | 1 | 11,557 | 218 |
| | 0.2 | 2,324 | 137 |
| PRO783 | 2 | 6,156 | 402 |
| | 0.5 | 2,803 | 314 |
| PRO419 | 1 | 4,300 | 236 |
| | 0.5 | 1,477 | NT |
| | 0.25 | 702 | NT |
| PRO 431 | 1 | 5,738 | 212 |
| | 0.5 | 2,161 | NT |
| | 0.25 | 1,100 | NT |

For the binding assays, RIA plates were coated with different amounts of purified anti-gp120 Ab3 for 18 hr with various amounts of antibody in PBS. The plates were blocked with 1% BSA in PBS, and 1 x $10^5$ cpm of $^{125}$I-labeled gp120$_{SF2}$ was added to each well. After 3 hr, the plates were washed and the radioactivities were measured. NT, not tested.

## Neutralizing Activity of gp120-Specific Ab3

Finally, Applicant examined the HIV-1 virus neutralizing activities of gp120-specific Ab3 antibodies. Table 6 summarizes the results. The gp 120-specific Ab3 from PRO703 neutralized MN, IIIB and RF strains of HIV-1. The gp120-specific Ab3 from PRO431 strongly neutralized the RF strain, and weakly neutralized tire Mn and IIIB strains. The gp120-specific Ab3 from PRO419 and PRO783 neutralized RF and MN, respectively. The lower or limited neutralizing activity of the gp120 specific Ab3s from PRO419, PRO431 and PRO783 may be due to lower affinity against certain gp120s. The gp120 binding activity of gp120-specific Ab3 from these monkeys was considerably lower than that of gp120-specific Ab3 from PRO703 (Table 5).

### Table 6.
**Neutralizing Activity of gp120-Specific Ab3 Antibodies Isolated From 3C9 Immune Primate Sera**

| Monkey | Ab3, ug/ml | Neutralizing activity as % inhibition of HIV isolates | | |
|---|---|---|---|---|
| | | HIV$_{MN}$ | HIV$_{IIIB}$ | HIV$_{RF}$ |
| PRO703 | 30 | 97 | 82 | 84 |
| | 10 | 97 | 20 | 94 |
| PRO783 | 55 | 82 | 0 | ND |
| | 18.8 | 8 | 0 | ND |
| PRO419 | 30 | 10 | 20 | 96 |
| | 15 | 8 | 0 | 98 |
| | 7.5 | 13 | 9 | 96 |
| PRO431 | 30 | 46 | 67 | 96 |
| | 15 | 17 | 56 | 98 |
| | 7.5 | 24 | 0 | 89 |

gp120-specific Ab3 antibodies produced by monkey PRO703 were isolated from a pool of sera obtained after the second, third, fifth, and sixth immunizations. gp120-specific Ab3 antibodies from monkey PRO783 were isolated from a pool of sera obtained after the second, third, fifth, sixth, and seventh immunizations. Ab3 antibodies from monkeys PRO419 and PRO431 were isolated from a pool of sera obtained after the fourth and fifth immunizations. The neutralizing activity was assessed by comparing the amount of p24 in the well of the test antibody with that in the control well. All experiments were performed in triplicate. ND, not done.

Collectively, the results from binding and virus neutralization studies on antibodies isolated from tire sera of 3C9-immunized non-human primates demonstrate that 3C9 can stimulate clones of primary B cells to produce broadly neutralizing HIV-1 antibodies.

## Claims

1. A method of selecting monoclonal anti-idiotype antibody reagents useful in vaccine formulations for the treatment or prevention of DIV infection, comprising the steps of:
   (a) preparing human polyclonal anti-gp120 antibodies (Ab1s);
   (b) generating a first plurality of monoclonal anti-idiotype antibodies (Ab2s) that immunologically binds with said human polyclonal anti-gp120 antibodies;
   (c) selecting a second plurality of monoclonal anti-idiotype antibodies from said first plurality of antibodies, wherein said second plurality of antibodies are characterized by their ability to immunologically bind with anti-gp120 antibodies that neutralize in vitro multiple strains of DIV; and
   (d) selecting a third plurality of monoclonal anti-idiotype antibodies from said second plurality of antibodies, wherein said third plurality of antibodies are characterized by their ability to generate an anti-anti-idiotype antibody (Ab3) response in a primate host, wherein said anti-anti-idiotype antibody immunologically binds with gp120 and neutralizes in vitro multiple strains of HIV.

2. The method of claim 1, wherein said human polyclonal anti-gp120 antibodies are pooled human sera from HIV positive, asymptomatic individuals.

3. The method of claim 2, wherein the said pooled human sera is purified for anti-gp120 antibodies by passing said pooled human sera over a column with gp120 bound to a stationary phase of said column and selecting, by elution, the bound fraction of anti-gp120 antibodies.

4. The method of any one of claims 1 to 3, wherein said first plurality of monoclonal anti-idiotype antibodies are murine.

5. The method of any one of claims 1 to 4, wherein said anti-gp120 antibodies to which the antibodies selected in step (c) can bind are human.

6. The method of claim 5, wherein said anti-gp120 antibodies to which the antibodies in step (c) can bind are human antibodies selected from said human polyclonal antibodies of step (a).

7. The method of claim 6, wherein said anti-gp120 antibodies to which the antibodies selected in step (c) can bind are selected by passing said human polyclonal antibodies of step (a) over a column with an anti-idiotype antibody from said first plurality of antibodies bound to a stationary phase of said column, eluting bound fraction of anti-gp120 antibodies and selecting from said bound fraction those anti-gp120 antibodies that are capable of neutralizing *in vitro* multiple strains of HIV.

8. A monoclonal anti-idiotype antibody reagent selected according to the method of any one of claims 1 to 7.

9. A monoclonal anti-idiotype that is capable of eliciting a group neutralizing anti-HIV antibody response in humans.

10. A hybridoma cell line capable of producing a monoclonal anti-idiotype antibody that is capable of eliciting a group neutralizing anti-HIV antibody response in humans.

11. A hybridoma cell line deposited with the ATCC, Rockville, MD, under accession No. HB 10701.

12. A monoclonal antibody produced by the cell line of claim 11.

13. A monoclonal antibody which elicits a substantially equivalent immune response in humans as a monoclonal antibody produced by said hybridoma cell line of claim 10 or 11.

14. A monoclonal antibody which reacts with and competes for binding to the same anti-gp120 antibody as a monoclonal antibody produced by the hybridoma cell line of claim 10 or 11.

15. A vaccine formulation comprising, in admixture with a pharmaceutically acceptable carrier, a monoclonal anti-idiotype antibody that is capable of eliciting a group neutralizing anti-HIV antibody response in humans.

16. A vaccine formulation comprising, in admixutre with a pharmaceutically acceptable carrier, an antibody of any one of claims 8, 9, 12, 13 or 14 in a dose sufficient to induce an active immune response in humans.

A pool of HIV+ sera

Affinity purification of Id+ Ab from serum.

3C10+ Ab
2D9+ Ab
702G+ Ab
702H+ Ab
702I+ Ab
4G11+ Ab
3C9+ Ab
4G5+ Ab
5A8+ Ab
5E8+ Ab
702J+ Ab

Characterize

Total anti-gp120 SF2 Ab

3C10 , 2D9 , 702G , 702H , 702I , 4G11 , 3C9 , 4G5 , 5A8 , 5E8 , 702J

A panel of monoclonal anti-idiotype antibodies

**Figure 1. Isolation of Id+ Anti-gp120 Abs.**

Figure 1A and 1B

Figure 2

Figure 3

**Total Anti-gp120 Abs**

gp120 - Sepharose

CD4 attachment site on gp120
blocked by sCD4

unbound fraction        bound fraction

applied to protein G column

bound fraction        unbound fraction (sCD4)

eluted with acidic buffer

anti-gp120 antibody
specific against
CD4 attachment site
(**CD4-site** Abs)

Figure 4

Figure 5

EP 0 503 916 A1

Figure 6

Figure 7

Characterization of the Corresponding Ab1s
Against Different Anti-id Mabs

Characteristics of Ab1s

| Ab2s | Ab1s | Binding to gp120SF2 | Binding to gp120IIIB | Virus Neutralization In | | |
|------|------|---------------------|----------------------|-------------------------|--------|---------|
| | | | | HIV$_{MN}$ | HIV$_{IIIB}$ | HIV$_{RF}$ |
| 3C10 | 3C10 id | + | - | +++ | · | - |
| 2D9 | 2D9 id | + | - | +++ | - | - |
| 1A12 | 1A12 id | + | - | + / - | - | - |
| 1C9 | 1C9 id | + | - | + | - | - |
| 3A6 | 3A6 id | + | + | +++ | + / - | - |
| 3C9 | 3C9 id | + | + | +++ | + + | +++ |
| 4G11 | 4G11 id | + | + | +++ | + / - | - |
| 2A12 | 2A12 id | + | - | +++ | - | - |
| 2F2 | 2F2 id | + | + | · | · | - |
| 4E10 | 4E10 id | + | + | ND | ND | ND |
| 4G5 | 4G5 id | + | + | +++ | +++ | +++ |
| 5A8 | 5A8 id | + | + | +++ | + + | - |
| 5E8 | 5E8 id | + | + | + + | + + | - |

*ND: Not done

Figure 8

## Characterization of the Corresponding Ab3s
## Against Different Ab2 in Monkeys

| | | | Characteristics of Ab3s | | |
|---|---|---|---|---|---|

| Ab2s | Ab3s | Binding to gp120 | Virus Neutralizations in | | |
|---|---|---|---|---|---|
| | | | HIV$_{MN}$ | HIV$_{IIIB}$ | HIV$_{RF}$ |
| 3C9 | 3C9 Ab3 | ++ (2/2)* | + | + | + |
| 4G11 | 4G11 Ab3 | - (0/2) | . | . | . |
| 4G5 | 4G5 Ab3 | + (2/2) | . | . | . |
| 5A8 | 5A8 Ab3 | - (0/2) | . | . | . |

*Number of positive responses from number of immunized monkeys in parenthesis

Figure 9

Neutralizing Activity of Ab3 Isolated From Monkey Sera

| Ab3 | Conc. of Ab | Neutralizing Activities as % Inhibition in | | |
|---|---|---|---|---|
| | | $HIV_{MN}$ | $HIV_{IIIB}$ | $HIV_{RF}$ |
| 3C9 Ab3* | 30 μg/ml | 97% | 82% | 84% |
| | 10 μg/ml | 97% | 20% | 94% |

*Purification was done on Ab2 and gp120 affinity column

Figure 10

EP 0 503 916 A1

Figure 11

Figure 12

EP 0 503 916 A1

## European Patent Office

# EUROPEAN SEARCH REPORT

Application Number

EP 92 30 2064

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | CANADIAN JOURNAL OF MICROBIOLOGY vol. 36, no. 11, 1 November 1990, MONTREAL CANADA pages 811 - 816; R.C. KENNEDY ET AL.: 'Concepts of idiotype-based vaccines for hepatitis b virus and human immunodeficiency virus.' * page 814, column 2, line 49 - page 815, column 2, line 17; figures 1,3 * | 1,4-14 | G01N33/569 G01N33/68 A61K39/21 C12P21/08 |
| Y | US-A-4 908 203 (G.B. THORTON ET AL.) * column 6, line 16 - column 8, line 5 * | 1-16 | |
| Y | AIDS RESEARCH AND HUMAN RETROVIRUSES vol. 6, no. 1, 1 January 1990, NEW YORK NY USA page 42; M.S.C. FUNG ET AL.: 'Anti-idiotype monoclonal antibodies to a neutralizing antibody (bat123) recognizing an immunodominant domain of hiv-1 gp120.' * the whole document * | 1-16 | TECHNICAL FIELDS SEARCHED (Int. Cl.5 ) G01N A61K |

The present search report has been drawn up for all claims

| Place of search | Date of completion of the search | Examiner |
|---|---|---|
| THE HAGUE | 23 JUNE 1992 | VAN BOHEMEN C.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P0401)

31